# EUROPEAN PATENT SPECIFICATION

(11) **EP 4 000 522 B1**
(45) Date of publication and mention of the grant of the patent: **14.02.2024**
(21) Application number: 21210165.3
(22) Date of filing: 24.11.2021
(51) Int. Cl.: A61B 5/20, A61B 5/145, A61B 5/00

(54) **SYSTEM FOR CONDUCTING URINALYSIS**
SYSTEM ZUR DURCHFÜHRUNG VON URINALYSEN
SYSTÈME POUR LA RÉALISATION D'ANALYSE D'URINE

(30) Priority: 24.11.2020 US 202063117464 P
(43) Date of publication of application: 25.05.2022
(73) Proprietor: Vivosens Inc, San Francisco, CA 94105 (US)
(72) Inventor: Tayfun, Miray, ISTANBUL (TR); Bengisu Karacaoglu, Balim, ISTANBUL (TR); Radman, George, MELBOURNE (AU)
(74) Representative: Cornuejols, Marine Sophie

(56) References cited:
- JP-A- S6 085 133
- US-A1- 2018 372 717
- US-A1- 2020 015 791
- PARK SEUNG-MIN ET AL: "A mountable toilet system for personalized health monitoring via the analysis of excreta", NATURE BIOMEDICAL ENGINEERING, NATURE PUBLISHING GROUP UK, LONDON, vol. 4, no. 6, 6 April 2020 (2020-04-06), pages 624-635, XP037524827, DOI: 10.1038/S41551-020-0534-9 [retrieved on 2020-04-06]

## Description

### FIELD OF INVENTION

The presently disclosed subject matter relates to a system for conducting urinalysis.

### BACKGROUND

Urinalysis is the physical, chemical, and microscopic examination of urine. It involves several tests to detect and measure various compounds that pass through the urine. When done on a frequent basis, urinalysis allows tracking changes in a person's body chemistry on a day-to-day basis.

PARK SEUNG-MIN ET AL: "A mountable toilet system for personalized health monitoring via the analysis of excreta", NATURE BIOMEDICAL ENGINEERING, NATURE PUBLISHING GROUP UK, LONDON, vol. 4, no. 6, 6 April 2020 (2020-04-06), pages 624-635, XP037524827,DOI: 10.1038/541551-020-0534-9, discloses a fully automated urinalysis module that can be mounted on existing toilets. A deck of test strips are loaded into a cartridge, which was designed as an insert into the toilet system. The beginning of urination is automatically detected with a passive infrared motion sensor. After motion is detected, a test strip is automatically loaded onto the deployment unit and strips are deployed using a unipolar stepper motor. After a sufficient amount of time for a user to fully saturate the strip with urine, the deployment unit is retracted into its original position, and then monitored by a video camera. After urination is complete, a strip-discarding servo motor attached to the deployment module releases the used strip such that the strip is discarded into the toilet bowl.

### SUMMARY OF INVENTION

There is provided in accordance with an aspect of the presently disclosed subject matter a system for conducting urinalysis of urine. The system includes a housing mountable on a urination device defining urination area; a dispenser mounted in the housing and being configured for holding a plurality of urine test strips (hereinafter "UTS"). Each one of the UTS includes at least one reacting area configured for a chemical reaction with a substance in the urine. The system further includes a detector configured for detecting at least one property of the reacting area and a shifting mechanism having a rotating arm configured for extracting one UTS from the dispenser and moving the UTS to a urination position in which the UTS is disposed above the urination area, such that the reacting area is disposed along a urine path.

The shifting mechanism is further configured for moving the rotating arm to a testing position in which the UTS is disposed at a location with respect to the detector facilitating thereby detection of the at least one property.

The urination device can be a seating toilet and the housing is a toilet seat.

The dispenser can be disposed on a first side of the perimeter of the urination area and the detector is disposed on a second side of the perimeter, and wherein the rotating arm is configured for holding the UTS and rotating the arm between the dispenser and the urination area.
The dispenser, the urination area and the detector can be disposed along a circular path, and wherein the shifting mechanism is configured for rotating the arm along the circular path.

The system can further include a waste compartment configured to hold used UTS, and wherein the shifting mechanism is configured to release used UTS into the waste compartment.

The system can further include an ejection aperture configured to allow access to used UTS, and wherein the shifting mechanism is configured to release used UTS into the ejection aperture.

The system can further include a controller for controlling operation of the shifting mechanism and the detector and a pressure sensor configured to detect when urine impinges upon the UTS, and wherein the controller is configured to control an amount of urine impinges upon the UTS.

The system can further include a disinfection device for cleaning the detector.

The UTS can include a holding portion and a reacting area, and wherein the holding portion has a narrower width than the width of the reacting area.
The UTS can be a colorimetric test strip and includes a plurality of pads each of which being configured for color change in response to reaction with the substance.

The at least one property can be a color of the pads and wherein the detector includes an array of sensors, each of which is configured a color in one of the pads.

The UTS can be a lateral flow test strip and wherein the reacting area includes a pad portion and an indicator portion, and wherein the shifting mechanism is configured to hold the UTS in the urination position, such that the pad portion is disposed along the urine path, and wherein the shifting mechanism is configured to hold the UTS in the testing position, such that the indicator portion is aligned with the detector and wherein the at least one property is a presence of the indicator.

The system can further include a communication module for communication with a remote IoT device and wherein the controller is configured to be controlled by the remote IoT device.

The detector can be configured to obtain an image of the UTS, and the communication module is configured to send the image to the remote IoT device.

The system of can further include a server in communication with the remote IoT device, and wherein the server is configured to receive urinalysis data of one urine test and determine kind and schedule for future urinalysis tests in accordance with the urinalysis data.

The UTS can be configured to detect at least one substance selected from the group consisting of calcium, UTIs, specific gravity, nitrites, blood, glucose, selenium, C peptide, chromium, pH, creatine, zinc, urobilinogen, bilirubin, leukocytes, microalbumin, urea, aldosterone total alkalinity, magnesium, potassium, hardness, iron, copper, lead, nitrate, fluoride, residual chlorine, breast cancer-specific antigen, prostate cancer-specific antigen, human chorionic gonadotropin, dopamine, serotonin, cortisol, luteinizing hormone, vitamin C, vitamin B12, vitamin B7, vitamin B9, heavy metals, estrogens, androgens, melatonin, progesterone, Testosterone, Sodium, chloride cortisone, Malondialdehyde, Peroxide, 8-OHdG free T3, free T4 drug abuse tests, and combinations thereof.

### BRIEF DESCRIPTION OF THE DRAWINGS

In order to understand the disclosure and to see how it may be carried out in practice, embodiments will now be described, by way of non-limiting examples only, with reference to the accompanying drawings, in which:
**Fig. 1** is a side perspective view of the system for conducting urinalysis in accordance with an example of the presently disclosed subject matter;
**Fig. 2A** is a partially exploded view of the system of Fig. 1 showing the shifting mechanism in a first position;
**Fig. 2B** is an enlarged view of the shifting mechanism of the system of Fig. 1;
**Fig. 3** is a partially exploded view of the system of Fig. 1 showing the shifting mechanism in a second position;
**Fig. 4** is a partially exploded view of the system of Fig. 1 showing the shifting mechanism in a third position;
**Fig. 5** is an upper view of the system for conducting urinalysis in accordance with another example of the presently disclosed subject matter;
**Fig. 6A** is an upper view of a urine test strip in accordance with an example of the presently disclosed subject matter;
**Fig. 6B** is an upper view of a urine test strip in accordance with another example of the presently disclosed subject matter; and
**Fig. 7** is a block diagram showing the disposition of the urine test strip of Fig. 6A, with respect to the detector.

### DETAILED DESCRIPTION OF EMBODIMENTS

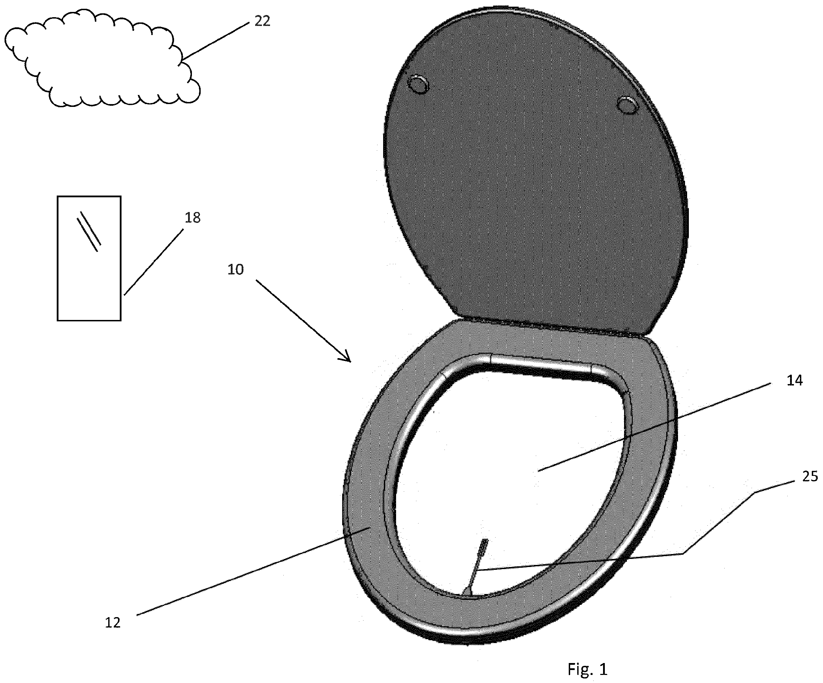

Figs. 1-3 show a system **10** for conducting urinalysis of urine by using a urine test strip disposed along the urine path. The system **10** includes a housing **12** mountable on a urination device (not shown) defining urination area **14.** According to the illustrated example, the urination device is a seating toilet, and the housing **12** is a toilet seat mounted on the toilet. According to other examples, the urination device can be a squat toilet, urinal, female urinal etc., and the housing can be a designated structure mounted on the urination device and defining a urination area **14.**

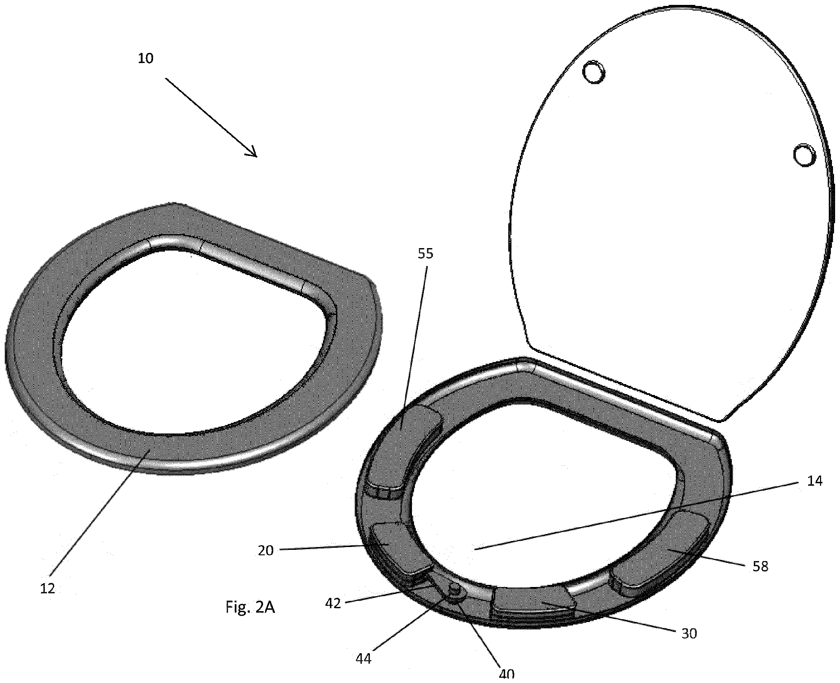

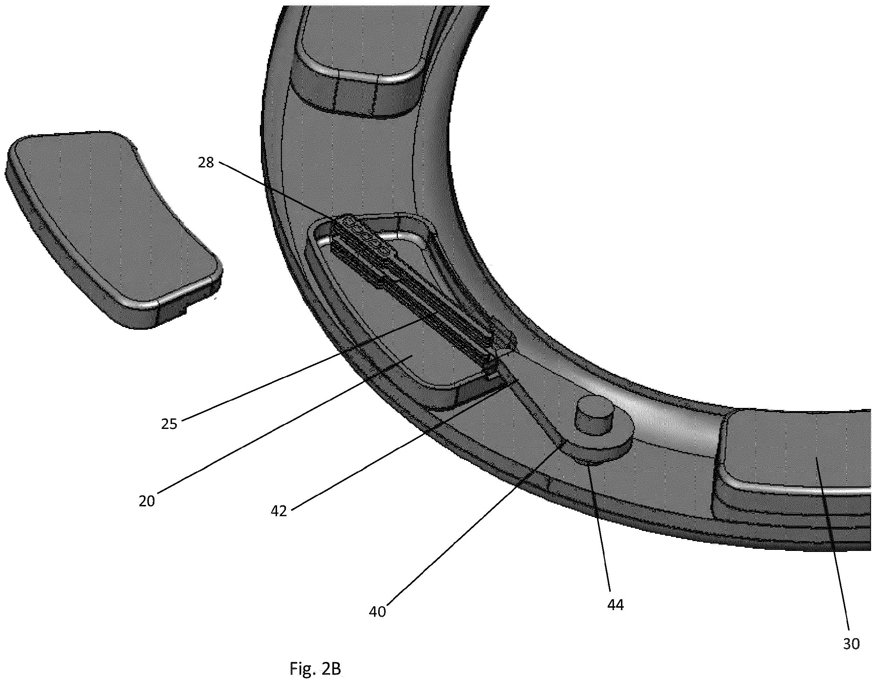

As shown in Figs. 2A and 2B, the housing **12** includes a dispenser **20,** which can be mounted inside the housing. The dispenser **20** is configured for holding a plurality of urine test strips (hereinafter "UTS") **25,** each of which having at least one reacting area **28** configured for a chemical reaction with a substance in the urine.

The UTS can be a lateral flow test having a reacting area **28** configured to detect the presence of a certain substance. Alternatively, the UTS can be a colorimetric test strip which includes one or more reacting area **28** in the form of pads. The reacting area **28** can be configured to react to various substances, such as calcium, UTIs, specific gravity, nitrites, blood, glucose, selenium, C peptide, chromium, pH, creatine, zinc, urobilinogen, bilirubin, leukocytes, microalbumin, urea, aldosterone total alkalinity, magnesium, potassium, hardness, iron, copper, lead, nitrate, fluoride, residual chlorine, breast cancer-specific antigen, prostate cancer-specific antigen, human chorionic gonadotropin, dopamine, serotonin, cortisol, luteinizing hormone, vitamin C, vitamin B12, vitamin B7, vitamin B9, heavy metals, estrogens, androgens, melatonin, progesterone, Testosterone, Sodium, chloride cortisone, Malondialdehyde, Peroxide, 8-OHdG free T3, free T4 drug abuse tests, and combinations thereof.

The housing **12** further includes a detector **30** configured for detecting at least one property of the reacting area **28.** For example, the detector can be an optical sensor configured to detect a certain feature of the reacting area **28.** For example, in case of lateral flow test, the detector **30** can be configured to detect the presence of an indicator on the reacting area **28,** such as one or more lines indicating the presence of a certain substance in the urine or the lack of such substance.

Alternatively, in the case of a colorimetric test strip, the detector **30** can be configured to detect a predetermined color, a number of colors, or gray level on the reacting area **28.** For example, the detector **30** can be configured to detect absorbance or can include a spectrophotometer for detecting certain colors. According to other examples, the detector **30** includes an imaging device configured for obtaining an image of the reacting area **28,** and an image processing apparatus for analyzing the obtained image. Analyzing the obtained image can be carried out by utilizing known methods of analyzing a urinalysis, for example the method disclosed in PCT publication WO2019221676.

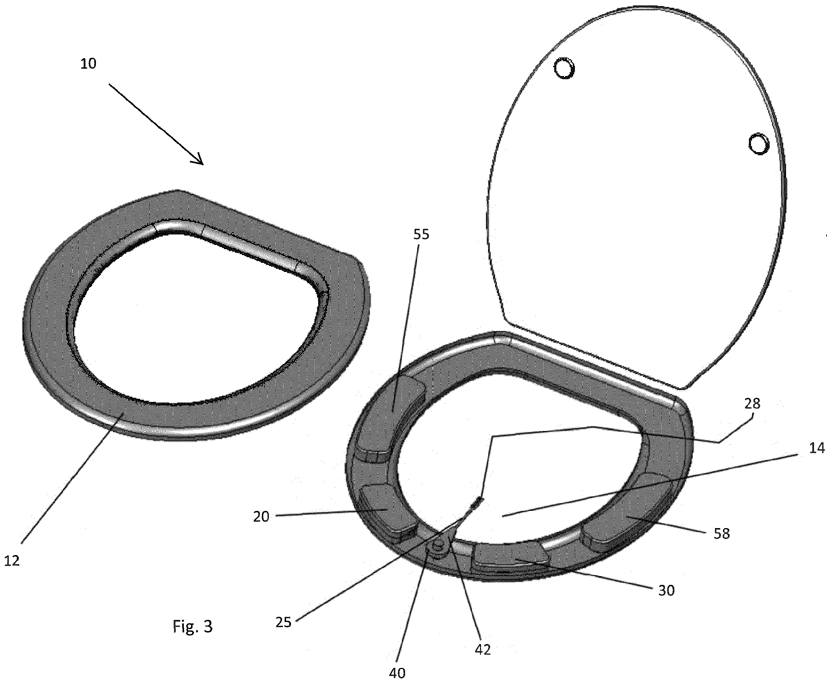

The system **10** further includes a shifting mechanism **40** configured for extracting one UTS **25** from the dispenser **20** and moving the UTS **25** to a urination position in which the UTS **25** is disposed above the urination area **14** such that the reacting area **28** is disposed along a urine path, as shown in Fig. 3.

The housing **12** can be provided with an opening (not shown) allowing removing of the dispenser **20,** such that the latter can be periodically replenished.

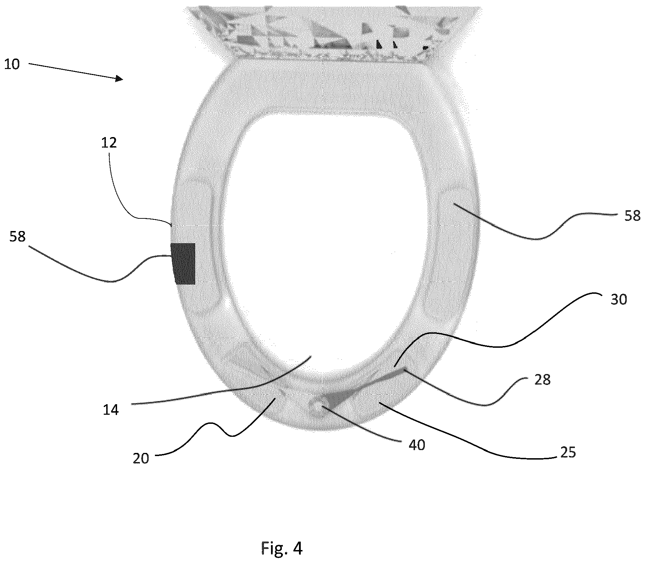

The shifting mechanism **40** can be further configured for moving the UTS **25** to a testing position in which the UTS **25** is disposed at a location with respect to the detector **30,** facilitating thereby detection of properties of the reacting area **28,** as described above. For example, the shifting mechanism **40** can be configured to shift the UTS **25** towards the detector **30** such that the reacting area **28** is disposed above the detector **30,** as shown in Fig. 4.

According to the illustrated example, the dispenser **20** is disposed on a first side of the perimeter of the urination area **14,** and the detector **30** is disposed on a second side of the perimeter of the urination area **14.** The shifting mechanism **40** includes an arm **42** configured for holding the UTS **25,** and the shifting mechanism includes a rotating motor **44** configured for rotating arm **42** between the dispenser **20** and urination area **14.** Similarly, the are **42** can be further rotated towards the detector **30,** shifting the UTS to the testing position, as shown in Fig. 4.

As shown in Fig. 2B, the dispenser **20** can include a slit allowing the arm **42** to enter the dispenser and grab one of the UTS **25** therein. The dispenser **20** can be provided with a spring mechanism urging the upper most UTS **25** to the slit facilitating thereby engagement of the arm **42** with the UTS **25.**

It would be appreciated by those skilled in the art that the shifting mechanism **40** may be configured to shift the UTS **25** from the dispenser **20** to the urination area **14** in other manners. For example, the dispenser **20** and the urination area **14** can be disposed along an axis and the shifting mechanism **40** can include a line conveyor configured to displace the UTS **25** from the dispenser **20** to the urination area **14.**

The shifting mechanism **40** is further configured to release the UTS **25** after the completion of the test and detection by the detector **30.** According to one example, the shifting mechanism **40** can be configured to rotate the UTS **25** back to the urination area **14** and to release the UTS **25** into the toilet. According to this example, the UTS 25 can be made from a biodegradable material or material which is decomposed and can be flashed in the toilet.

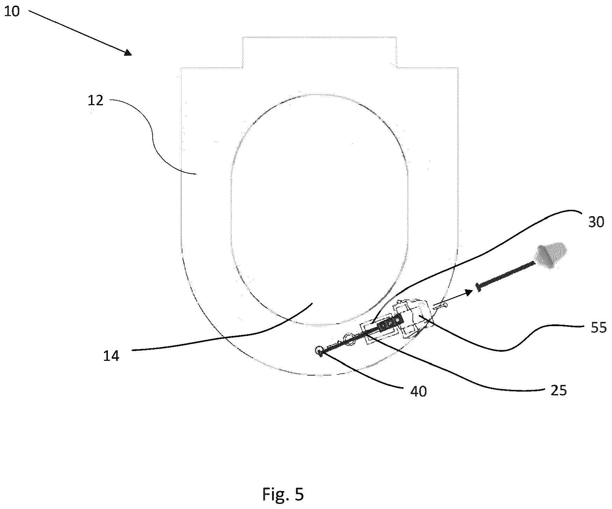

According to another example, as shown in Fig. 5, the system **10** can include a waste compartment **50** configured to hold a plurality of used UTSs **25.** The waste compartment **50** can be located in close proximity to the detector **30,** and the shifting mechanism **40** can be configured to shift the used UTS **25** into the waste compartment **50.** According to an example, the waste compartment **50** is disposed along a straight line from the detector **30,** such that when the shifting mechanism **40** releases the UTS **25,** the latter falls into the waste compartment **50.**

Alternatively, the detector **30** and the waste compartment **50** can be disposed along the same rotational path, such that the UTS is first shifted to the testing position above the detector **30.** Further shifting of the UTS **25** along the same rotational path brings the latter into the waste compartment **50,** where it can be released. The housing can be provided with an opening allowing removing of the waste compartment **50,** such that the latter can be periodically emptied.

According to a further example, the housing **12** can include an ejection aperture (not shown) defined in close proximity of the detector **30.** Upon completion of the test and detection of the UTS **25,** the shifting mechanism **40** releases the UTS **25** into the ejection aperture, allowing the user to remove the used UTS **25.**

The housing **12** further includes a power source, such as a battery **55,** and a controller **58** for controlling the operation of the shifting mechanism **40** and the detector **30.** The controller **58** may include a pressure sensor configured to detect when urine impinges upon the UTS **25.** The controller **58** can be configured to control the amount of urine that reaches the UTS **25,** for example, by initiating the shifting of the UTS **25** towards the detector **30** after a predetermined time since the urination starts. Alternatively, the pressure sensor can be utilized for detecting when urination stopped and to assess whether or not a sufficient amount of urine has reached the UTS **25.**

The controller **58** can further include a processor for processing detection data received from the detector **30** and to determine health data in accordance with the detection data. According to an example, the controller **58** can further include a communication module, such as WiFi or Bluetooth transceiver, etc., for transmitting the detection or health data to a remote server **22** (as shown in Fig. 1), as explained herein after.

According to an example, the housing **12** may be provided with a disinfection device, such as, UV light bulb for cleaning the detector **30,** or the ejection aperture, as described above.

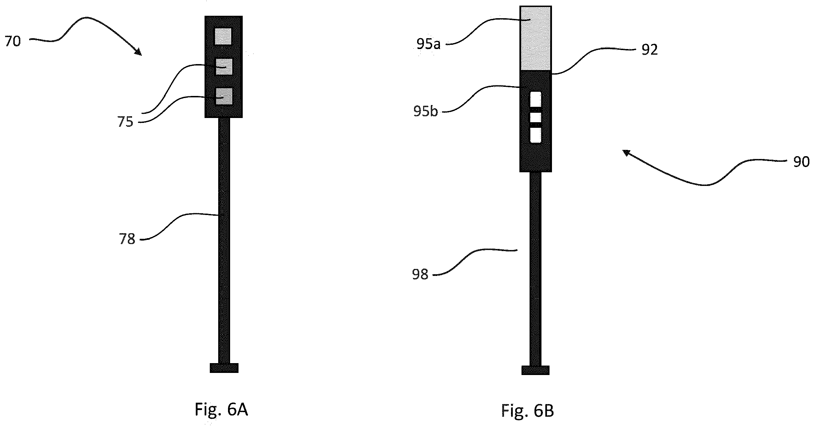

As shown in Fig. 6A, the UTS can be a colorimetric test strip **70** having a reacting area in the form of three or more pads **75,** and a holding portion **78.** The holding portion **78** is configured to be grasped by the shifting mechanism **40.** As shown in the present example, the holding portion **78** is narrower relative to the reacting area, minimizing thereby the amount of urine impinging upon the holding portion **78.** This way, the amount of urine entering areas around the detector **30** is reduced. According to this example, the urine reacts with the pads and causes the color of the pads to change depending on the presence of certain substances in the urine.

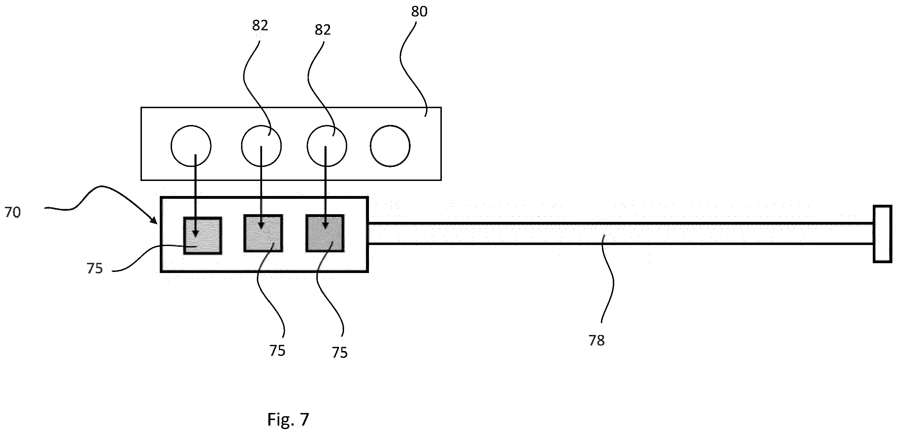

According to an example, each of the pads **75** can be configured to detect a different substance and to provide an indication with a predetermined color. Accordingly, as shown in Fig 7, the detector **80** can be configured to detect each of the colors corresponding to the colors indicated by the pads **75.** For example, the detector **80** can include an array of sensors **82,** each of which is configured to detect one color. Thus, according to this example, the shifting mechanism **40** is configured to shift the UTS **70** towards the detector **80** such that the position of each of the pads **75** is aligned with the corresponding sensor **82.**

According to another example, the UTS **90** can be a lateral flow test strip having a holding portion **98** and a reacting area **92.** According to this example, reacting area **92** includes a pad portion **95a** and an indicator portion **95b.** Thus, the shifting mechanism **40** is configured such that when the UTS **90** is in the urination position, the pad portion **95a** is disposed along the urine path, allowing the urine to react with the pad portion **95a.** However, when the UTS **90** is shitted to the testing position, the shifting mechanism **40** is configured to dispose the UTS **90** such that the indicator portion **95b** is aligned with the detector.

As shown in Fig. 1, according to an example, the system can be connected with IoT (Internet Of Things) devices **18,** such as smartphones, watches, or other devices. According to this example, the detector can be configured to obtain an image of the UTS and data of the image is sent to an external IoT device **18.** The external IoT device can include a microprocessor for processing the captured images and determining the results of the test. In some embodiments, the urinalysis system, which is connected to IoT devices (e.g. Smartphones) is configured to allows the user to control and operate the urinalysis system using such devices wirelessly. Alternatively, the urinalysis system can be operated using physical buttons that may be attached to any part of the toilet seat.

According to an example, the IoT device **18** can include a designated application with a user interface allowing the user to control various features of the system 10. For example, IoT device **18** can be configured to allow the user to alter the position of the arm **42.** The arm **42** can be moved by the user to the appropriate location depending on the user's gender, height or preferences.

According to an example, a user can initiate a urinalysis test by clicking on the appropriate command or button on the manual button of a remote or IoT device **18.** Alternatively, the IoT device can be configured to monitor the urine on a regular basis and provide the user with plans and wellness advice. Similarly, the urinalysis system can be configured for performing automated or manual tests depending upon the input of the user. Furthermore, the IoT device **18** can be connected to a server **22** which collects test data and provides medical recommendations in accordance with the results. The server **22** and the IoT device **18** can thus provide a testing schedule depending on the data received from the tests. In other words, in case data from a test indicates the existence of a certain substance in the urine, or the lack of a certain substance, the system server **22** and the IoT device **18** can be configured to determine a specific schedule for further tests, which can include specific time of the day, frequency of tests and or the kind of test required for monitoring health of the user. It would be appreciated that the toilet seat containing the urinalysis apparatus can be mounted on commonly available toilet bowls in the market.

It is noted that the known toilets that are capable of performing urinalysis are complex to build and use. One of the major problems with the existing smart toilets or toilet bowls is that they require a separate urine collection chamber. As a result, a toilet has to be redesigned and rebuilt or at the least, the toilet bowl has to be replaced with a new smart toilet bowl. The process of redesigning and rebuilding toilets is expensive. Hence, it is difficult to rebuild the toilets at a large scale. Further, cleaning such toilets with a urine collection chamber proves to be a difficult task for users. The existing smart toilets are also hard to operate.

The present invention aims to solve this problem. The present invention can be easily be integrated into most of the toilet bowls in the market. The present invention has an adjustable urination structure. The invention is easy to operate since it can be operated with IoT (Internet of Things) devices such as mobile phones, tabs, smartwatches, etc. The process is unlike the hospital or lab tests or manual at-home tests. The testing process is fully automated and the best part being, that no laboratory or clinical know-how is needed to perform or to analyze the test. The results can be analyzed and monitored on a mobile device. This will require no pre-requisite knowledge of technical know-how or clinical assistance for the user. With the cartilage disposing mechanism designed in the invention, urine strip waste can be disposed hygienically after the procedure.

Those skilled in the art to which the presently disclosed subject matter pertains will readily appreciate that numerous changes, variations, and modifications can be made without departing from the scope of the invention, *mutatis mutandis.*

## Claims

1. A system for conducting urinalysis of urine comprising:
a housing mountable on a urination device defining urination area;
a dispenser mounted in said housing and being configured for holding a plurality of urine test strips (hereinafter "UTS"), each one of said UTS includes at least one reacting area configured for a chemical reaction with a substance in the urine;
a detector configured for detecting at least one property of said reacting area;
the system **characterized in that** it comprises a shifting mechanism having a rotating arm configured for extracting one UTS from said dispenser and moving said UTS to a urination position in which said UTS is disposed above said urination area, such that said reacting area is disposed along a urine path;
said shifting mechanism is further configured for moving said rotating arm to a testing position in which said UTS is disposed at a location with respect to said detector facilitating thereby detection of said at least one property.

2. The system according to claim 1, wherein the dispenser is disposed on a first side of the perimeter of the urination area and the detector is disposed on a second side of the perimeter, and wherein said rotating arm is configured for holding said UTS and rotating said arm between said dispenser and said urination area.

3. The system according to claim 2, wherein the dispenser, the urination area and the detector are disposed along a circular path, and wherein said shifting mechanism is configured for rotating said arm along said circular path.

4. The system according to one of claims 1 to 3, further comprising a waste compartment configured to hold used UTS, and wherein said shifting mechanism is configured to release used UTS into said waste compartment.

5. The system according to one of claims 1 to 4, further comprising a controller for controlling operation of said shifting mechanism and said detector and a pressure sensor configured to detect when urine impinges upon said UTS, and wherein said controller is configured to control an amount of urine impinges upon said UTS.

6. The system according to one of claims 1 to 5, further comprising a disinfection device for cleaning said detector.

7. The system according to one of claims 1 to 4, wherein said UTS includes a holding portion and a reacting area, and wherein said holding portion has a narrower width than the width of said reacting area.

8. The system according to claim 7, wherein said UTS is a colorimetric test strip and includes a plurality of pads each of which being configured for color change in response to reaction with said substance.

9. The system according to claim 8 wherein said at least one property is a color of said pads and wherein said detector includes an array of sensors, each of which is configured a color in one of said pads.

10. The system according to one of claims 7 to 9, wherein said UTS is a lateral flow test strip and wherein said reacting area includes a pad portion and an indicator portion, and wherein said shifting mechanism is configured to hold said UTS in said urination position, such that said pad portion is disposed along said urine path, and wherein said shifting mechanism is configured to hold said UTS in said testing position, such that said indicator portion is aligned with said detector and wherein said at least one property is a presence of said indicator.

11. The system according to claim 5, further comprising a communication module for communication with a remote IoT device and wherein said controller is configured to be controlled by said remote IoT device.

12. The system according to claim 11, wherein said detector is configured to obtain an image of said UTS, and said communication module is configured to send said image to said remote IoT device.

13. The system according to claim 11 or 12, further comprising a server in communication with said remote IoT device, and wherein said server is configured to receive urinalysis data of one urine test and determine kind and schedule for future urinalysis tests in accordance with said urinalysis data.

14. The system according to one of claims 1 to 13, wherein said UTS is configured to detect at least one substance selected from the group consisting of calcium, UTIs, specific gravity, nitrites, blood, glucose, selenium, C peptide, chromium, pH, creatine, zinc, urobilinogen, bilirubin, leukocytes, microalbumin, urea, aldosterone total alkalinity, magnesium, potassium, hardness, iron, copper, lead, nitrate, fluoride, residual chlorine, breast cancer-specific antigen, prostate cancer-specific antigen, human chorionic gonadotropin, dopamine, serotonin, cortisol, luteinizing hormone, vitamin C, vitamin B12, vitamin B7, vitamin B9, heavy metals, estrogens, androgens, melatonin, progesterone, Testosterone, Sodium, chloride cortisone, Malondialdehyde, Peroxide, 8-OHdG free T3, free T4 drug abuse tests, and combinations thereof.

## Patentansprüche

1. Ein System zur Durchführung von Urinanalysen, umfassend:
ein Gehäuse, das an eine Urinierhilfe montiert werden kann, das den Urinierbereich definiert;
ein im Gehäuse montierter und zum Halten mehrerer
Urinteststreifen (nachfolgend "UTS") konfigurierter Spender, wobei jeder dieser UTS mindestens einen Reaktionsbereich umfasst, der für eine chemische Reaktion mit einer Substanz im Urin ausgelegt ist;
einen Detektor, der so eingerichtet ist, dass er wenigstens eine Eigenschaft des genannten Reaktionsbereichs erkennt;
das System ist **dadurch gekennzeichnet, dass** es einen Verschiebemechanismus mit einem Dreharm umfasst, der so eingerichtet ist, dass er ein einen UTS aus dem Spender entnimmt und den UTS in eine Urinierposition bewegt, in welcher der UTS so über dem Urinierbereich angeordnet wird, dass der Reaktionsbereich entlang eines Urinpfads angeordnet ist;
wobei dieser Verschiebemechanismus ferner zum Bewegen des Dreharms in eine Prüfposition eingerichtet ist, in der der UTS in Bezug auf den Detektor an einer Stelle angeordnet ist, in der die Erkennung der wenigstens einen Eigenschaft erleichtert wird.

2. System nach Anspruch 1, wobei der Spender auf einer ersten Seite des Perimeters des Urinierbereichs und der Detektor auf einer zweiten Seite des Perimeters angeordnet ist, und wobei der Dreharm Z zum Halten des UTS und Drehen des Arms zwischen dem Spender und dem Urinierbereich eingerichtet ist.

3. System nach Anspruch 2, wobei der Spender, der Urinierbereich und der Detektor entlang eines kreisförmigen Pfades angeordnet sind, und wobei der Verschiebemechanismus zum Drehen des Arms entlang des kreisförmigen Pfades eingerichtet ist.

4. System nach einem der Ansprüche 1 bis 3, das ferner ein Abfallfach umfasst, das so eingerichtet ist, dass es gebrauchte UTS aufnimmt, und wobei der Verschiebemechanismus so eingerichtet ist, dass er gebrauchte UTS in das Abfallfach freigibt.

5. System nach einem der Ansprüche 1 bis 4, ferner umfassend ein Steuergerät zur Steuerung des Betriebs des Verschiebemechanismus und des Detektors sowie einen Drucksensor, der so eingerichtet ist, dass er erkennt, wenn Urin auf den UTS auftrifft, und wobei das Steuergerät so eingerichtet ist, dass es den Aufprall einer Urinmenge auf den UTS steuert.

6. System nach einem der Ansprüche 1 bis 5, ferner umfassend eine Desinfektionsvorrichtung zur Reinigung des Detektors.

7. System nach einem der Ansprüche 1 bis 4, wobei der UTS einen Halteabschnitt und einen Reaktionsbereich umfasst, und wobei die Breite des Halteabschnitts schmaler ist als die Breite des Reaktionsbereichs.

8. System nach Anspruch 7, wobei UTS ein kolorimetrischer Teststreifen ist und eine Vielzahl von Pads umfasst, die jeweils für eine Farbänderung als Reaktion mit der besagten Substanz eingerichtet sind.

9. System nach Anspruch 8, wobei die wenigstens eine Eigenschaft eine Farbe der Pads ist und wobei der Detektor eine Anordnung von Sensoren umfasst, von denen jeder als eine Farbe in einem der Pads eingerichtet ist.

10. System nach einem der Ansprüche 7 bis 9, wobei der UTS ein seitlicher Durchflussteststreifen ist und wobei der Reaktionsbereich einen Padteil und einen Indikatorteil umfasst, und wobei der Verschiebemechanismus so eingerichtet ist, dass er den UTS so in der Urinierposition hält, dass der Padteil entlang des Urinpfads liegt, und wobei der Verschiebemechanismus so eingerichtet ist, dass er den UTS so in der Testposition hält, dass der Indikatorteil mit dem Detektor fluchtet und wobei die wenigstens eine Eigenschaft das Vorhandensein des Indikators darstellt.

11. System nach Anspruch 5, ferner umfassend ein Kommunikationsmodul zur Kommunikation mit einer entfernten IoT-Gerät und wobei das Steuergerät so eingerichtet ist, dass es von dem entfernten IoT-Gerät gesteuert wird.

12. System nach Anspruch 11, wobei der Detektor so eingerichtet ist, dass er ein Bild des UTS erhält, und das Kommunikationsmodul so eingerichtet ist, dass es das Bild an das entfernte IoT-Gerät sendet.

13. System nach Anspruch 11 oder 12, ferner umfassend einen Server, der mit dem entfernten IoT-Gerät kommuniziert, und wobei der Server so eingerichtet ist, dass er Urinanalysedaten eines Urintests empfängt und Art und Zeitplan für zukünftige Urinanalysetests gemäß den Urinanalysedaten bestimmt.

14. System nach einem der Ansprüche 1 bis 13, wobei der UTS so eingerichtet ist, dass er wenigstens eine Substanz erkennt, die aus der Gruppe ausgewählt ist, die aus Kalzium, Harnwegsinfektionen, spezifischem Gewicht, Nitriten, Blut, Glukose, Selen, C-Peptid, Chrom, pH, Kreatin, Zink, Urobilinogen, Bilirubin, Leukozyten, Mikroalbumin, Harnstoff, Aldosteron-Gesamtalkalinität, Magnesium, Kalium, Härte, Eisen, Kupfer, Blei, Nitrat, Fluorid, Restchlor, Brustkrebs-spezifisches Antigen, Prostatakrebs-spezifisches Antigen, humanes Choriongonadotropin, Dopamin, Serotonin, Kortisol, luteinisierendes Hormon, Vitamin C, Vitamin B12, Vitamin B7, Vitamin B9, Schwermetalle, Östrogene, Androgene, Melatonin, Progesteron, Testosteron, Natrium, Chloridkortison, Malondialdehyd, Peroxid, 8- OHdG freies T3, freies T4 Drogenmissbrauchstests und Kombinationen davon besteht.

## Revendications

1. Système pour effectuer une analyse d'urine comprenant :
un boîtier montable sur un dispositif de miction définissant une zone de miction ;
un distributeur monté dans ledit boîtier et étant configuré pour maintenir une pluralité
de bandelettes urinaires (ci-après « UTS »), chacune desdites UTS inclut au moins une zone de réaction configurée pour une réaction chimique avec une substance dans l'urine ;
un détecteur configuré pour détecter au moins une propriété de ladite zone de réaction ;
le système étant **caractérisé en ce qu'**il comprend un mécanisme de déplacement ayant un bras rotatif configuré pour extraire une UTS dudit distributeur et mouvoir ladite UTS vers une position de miction dans laquelle ladite UTS est disposée au-dessus de ladite zone de miction, de sorte que ladite zone réactive est disposée le long d'une voie urinaire ;
ledit mécanisme de déplacement est en outre configuré pour mouvoir ledit bras rotatif vers une position de test dans laquelle ladite UTS est disposée à un endroit par rapport audit détecteur facilitant ainsi la détection de ladite au moins une propriété.

2. Système selon la revendication 1, dans lequel le distributeur est disposé sur un premier côté du périmètre de la zone de miction et le détecteur est disposé sur un second côté du périmètre, et dans lequel ledit bras rotatif est configuré en Z pour maintenir ladite UTS et faire tourner ledit bras entre ledit distributeur et ladite zone de miction.

3. Système selon la revendication 2, dans lequel le distributeur, la zone de miction et le détecteur sont disposés le long d'une voie circulaire, et dans lequel ledit mécanisme de déplacement est configuré pour faire tourner ledit bras le long de ladite voie circulaire.

4. Système selon l'une des revendications 1 à 3, comprenant en outre un compartiment à déchets configuré pour maintenir une UTS usagée, et dans lequel ledit mécanisme de déplacement est configuré pour libérer une UTS usagée dans ledit compartiment à déchets.

5. Système selon l'une des revendications 1 à 4, comprenant en outre un contrôleur pour contrôler le fonctionnement dudit mécanisme de déplacement et dudit détecteur et un capteur de pression configuré pour détecter quand l'urine arrive sur ladite UTS, et dans lequel ledit contrôleur est configuré pour contrôler une quantité d'urine qui arrive sur ladite UTS.

6. Système selon l'une des revendications 1 à 5, comprenant en outre un dispositif de désinfection pour le nettoyage dudit détecteur.

7. Système selon l'une des revendications 1 à 4, dans lequel ladite UTS inclut une portion de maintien et une zone de réaction, et dans lequel ladite portion de maintien a une plus petite largeur que la largeur de ladite zone de réaction.

8. Système selon la revendication 7, dans lequel ladite UTS est une bandelette de test colorimétrique et
inclut une pluralité de tampons dont chacun est configuré pour changer de couleur en réponse à une réaction avec ladite substance.

9. Système selon la revendication 8, dans lequel ladite au moins une propriété est une couleur desdits tampons et dans lequel ledit détecteur inclut un ensemble de capteurs, dont chacun est configuré avec une couleur dans l'un desdits tampons.

10. Système selon l'une des revendications 7 à 9, dans lequel ladite UTS est une bandelette de test à flux latéral et dans lequel ladite zone de réaction comprend une portion de tampon et une portion d'indicateur, et dans lequel ledit mécanisme de déplacement est configuré pour maintenir ladite UTS dans ladite position de miction, de sorte que ladite portion de tampon est disposée le long de ladite voie urinaire, et dans lequel ledit mécanisme de déplacement est configuré pour maintenir ladite UTS dans ladite position de test, de sorte que ladite portion d'indicateur est alignée avec ledit détecteur et dans laquelle ladite au moins une propriété est une présence dudit indicateur.

11. Système selon la revendication 5, comprenant en outre un module de communication pour une communication avec un dispositif IoT distant et dans lequel ledit contrôleur est configuré pour être contrôlé par ledit dispositif IoT distant.

12. Système selon la revendication 11, dans lequel ledit détecteur est configuré pour obtenir une image de ladite UTS, et ledit module de communication est configuré pour envoyer ladite image audit dispositif IoT distant.

13. Système selon la revendication 11 ou 12, comprenant en outre un serveur en communication avec ledit dispositif IoT distant, et dans lequel ledit serveur est configuré pour recevoir des données d'analyse d'urine d'un test d'urine et déterminer la sorte et le programme des futurs tests d'analyse d'urine conformément auxdites données d'analyse d'urine.

14. Système selon l'une des revendications 1 à 13, dans lequel ladite UTS est configurée pour détecter au moins une substance choisie dans le groupe consistant en calcium, IVU, densité, nitrites, sang, glucose, sélénium, peptide C, chrome, pH, créatine, zinc, urobilinogène, bilirubine, leucocytes, microalbumine, urée, titre alcalin total d'aldostérone, magnésium, potassium, dureté, fer, cuivre, plomb, nitrate, fluorure, chlore résiduel, antigène spécifique du cancer du sein, antigène spécifique du cancer de la prostate, gonadotrophine chorionique humaine, dopamine, sérotonine, cortisol, hormone lutéinisante, vitamine C, vitamine B12, vitamine B7, vitamine B9, métaux lourds, oestrogènes, androgènes, mélatonine, progestérone, testostérone, sodium, chlorure de cortisone, malondialdéhyde, peroxyde, T3 libre 8- OHdG, tests d'abus de drogues T4 libre et leurs combinaisons.
